# EUROPÄISCHE PATENTANMELDUNG

(11) **EP 2 444 425 A1**
(43) Veröffentlichungstag der Anmeldung: **25.04.2012**
(21) Anmeldenummer: 11184320.7
(22) Anmeldetag: 07.10.2011
(51) Int. Cl.: C08B 37/00, A61K 9/20, A61K 31/02, A61K 31/08, A61K 47/48

(54) **1:1, 2:1- oder 3:1-Komplex bestehend aus einem Cyclodextrin oder Cyclodextrinderivat und einem halogenierten Ether, seine Herstellung und seine Verwendung als Schlafmittel**

(30) Priorität: 19.10.2010 DE 102010042615
(71) Anmelder: Wacker Chemie AG, 81737 München (DE)
(72) Erfinder: Busold, Markus, 81825 München (DE); Mitterweger, Simone, 82205 Gilching (DE); Regiert, Maria-Elisabeth, 80538 München (DE); Sigl, Harald, 84513 Töging (DE)
(74) Vertreter: Potten, Holger

(57) **Zusammenfassung**

Die vorliegende Erfindung betrifft einen Komplex bestehend aus einem Cyclodextrin oder Cyclodextrinderivat und einem niedermolekularen halogeniertem Ether im molaren Verhältnis 1:1 oder 2:1 oder 3:1.

## Beschreibung

Die Erfindung betrifft einen 1:1, 2:1 oder 3:1- Komplex (Cyclodextrin bzw. Cyclodextrinderivat : halogeniertem Ether) bestehend aus einem Cyclodextrin oder Cyclodextrinderivat und einem niedermolekularen halogeniertem Ether, seine Herstellung und seine Verwendung als Schlafmittel.

Cyclodextrine sind cyclische Oligosaccharide, die aus 6, 7 oder 8 (1-4)-verknüpften Anhydroglukoseeinheiten aufgebaut sind. Die durch enzymatische Stärkekonversion hergestellten alpha-, beta- oder gamma-Cyclodextrine unterscheiden sich im Durchmesser ihrer hydrophoben Kavität und eignen sich generell zum Einschluß zahlreicher lipophiler Substanzen.

Narkosemittel bzw. Anästhetika dienen zum medikamentösen Herbeiführen eines kontrollierten Zustandes der Bewusstlosigkeit auch genannt Narkose oder Anästhesie. Dabei werden im gesamten Körper durch Lähmung des zentralen Nervensystems neben dem Bewusstsein sowohl die Schmerzempfindung, die Abwehrreflexe als auch die Muskelspannung abgeschaltet.

Eine wissenschaftliche Beschreibung der Narkose lautet "pharmakologisch induziertes, reversibles Koma". Die künstlich herbeigeführte Narkose bewirkt somit die zeitweilige, umkehrbare Funktionshemmung des zentralen Nervensystems (ZNS) mit Herbeiführung von Bewusstseinsverlust (Schlaf) und Ausschaltung des Schmerzempfindens (Analgesie). In vielen Fällen ist zusätzlich die schlaffe Lähmung der Willkür-Muskulatur erwünscht. Die Narkose geht mit einer Dämpfung der Reflexe einher.

Niedermolekulare halogenierte Ether wie Sevofluran (1,1,1,3,3,3-Hexafluor-2-(fluormethoxy)propan), Isofluran (2-Chloro-2-(Difluoromethoxy)-1,1,1-Trifluorethan sowie Desfluran (2-(Bifluormethoxy)-1,1,1,2-Tetrafluorethan) werden als Narkosemittel eingesetzt.

Sevofluran ist der internationale Freiname für das Inhalationsnarkotikum 1,1,1,3,3,3-Hexafluor-2-(fluormethoxy)-propan) mit der Summenformel C₄H₃F₇O. Sevofluran ist ein Inhalationsnarkotikum und zeichnet sich durch eine rasche Kinetik aus (rasches An- und Abfluten). Im Gegensatz zu anderen Inhalationsnarkotika weist es keinen beißenden Geruch auf und führt nicht zu Irritationen der Atemwege oder Beeinträchtigung der Nieren- oder Herzfunktion. Es ist daher eine gute Alternative für die Einleitung bei Kindernarkosen. Es ist zudem kein Gefahrstoff. Sevofluran ist unter der Bezeichnung Sevorane^{®} (Abbott) im Handel erhältlich. Sevofluran weist einen Siedepunkt von 59°C bei Normaldruck (1bar) auf und ist leicht flüchtig. Die Löslichkeit in Wasser beträgt 0,15g/100g.

Isofluran ist der internationale Freiname für das Inhalationsnarkotikum (2-Chloro-2-(Difluoromethoxy)-1,1,1-Trifluorethan mit der Summenformel C₃H₂ClF₅O. Isofluran ist ein Inhalationsnarkotikum, welches neben der Anwendung beim Menschen auch im Veterinärsektor Verwendung findet.

Isofluran weist einen Siedepunkt von 49°C bei Normaldruck auf und ist leicht flüchtig.

Desfluran ist der internationale Freiname für das Inhalationsnarkotikum (2-(Bifluormethoxy)-1,1,1,2-Tetrafluorethan) mit der Summenformel C₃H₂F₆O.

Desfluran weist einen noch niedrigeren Siedepunkt als die vorgenannten Verbindungen mit 24°C bei Normaldruck auf und ist sehr leicht flüchtig. Es hat eine gute hypnotische, jedoch nur schwache analgetische und muskelrelaxierende Wirkung. Desfluran ist ein in der Anästhesie weit verbreitetes Standardanästhetikum. Es zeichnet sich durch schnelles An- und Abfluten und damit gute Steuerbarkeit sowie eine gute Kreislaufstabilität aus. In Deutschland, Österreich und der Schweiz wird es von Baxter unter dem Handelsnamen Suprane^{®} vertrieben.

Wegen dieser Flüchtigkeit ist der von Sevofluran, Isofluran und Desfluran erwünschte breite Einsatz in u. a. flüssigen wie festen pharmazeutischen Produkten, wie Einschlafmittel, nicht möglich. Des Weiteren ist die kontrollierte Freisetzung aus einer wässrigen Zubereitung von Cyclodextrinen wünschenswert.

Die Anmeldung DE4317139 beschreibt ein Verfahren zur präparativen Enantiomerentrennung von chiralen Inhalationsanästhetika wie racemischem Enfluranen, also Fluorkohlenwasserstoffen, z.B. Sevofluran, mittels an Chromosorb P gebundenem octakis(2,6-0-di-n-pentyl-3-0-butanoyl)-gamma Cyclodextrin.

WO2009094460 zitiert eine Lösung bzw. Emulsion einer flüchtigen Anesthetika-Komposition von Fluorkohlenwasserstoffen, z.B. Sevofluran basierend bevorzugt auf einem organischen Lösungsmittel wie DMSO oder NMP zur Reduktion der Flüchtigkeit der Anesthetika, sowie die Anwendung von Cyclodextrinen ganz allgemein als Lösungsvermittler. Cyclodextrinkomplexe werden nicht genannt, der Einsatz des Cyclodextrins wird nur ganz allgemein und unbestimmt erwähnt, es handelt sich nur um eine reine Zugabe und nicht um einen Komplexierungsprozess.

US6106858 beschreibt die Ladung von Wirkstoffen wie Anesthetika in multivascularen Liposomen zur Regulierung der Osmolarität bei einem eventuellen Zusatz von unkomplexierten Cyclodextrinen. Der Einsatz des Cyclodextrins wird nur ganz allgemein und unbestimmt erwähnt, es handelt sich nur um eine reine Zugabe und nicht um einen Komplexierungsprozess.

WO0119366 beschreibt Oxazolidonon in Kombination mit unkomplexierten Cyclodextrinen als Löslichkeitsverstärker und einem zusätzlichen Medikament (z.B. Anästhetika allgemein) zur topischen Behandlung bzw. zum Schutz von ocularen Infektionen. Die Zugabe des Cyclodextrins erfolgt undefiniert und ohne spezifische Verfahren, es handelt sich nicht um einen Komplexierungsprozess.

V. Schurig and R. Schmidt, "Extraordinary chiral discrimination in inclusion gas chromatography. Thermodynamics of enantioselectivity between a racemic perfluorodiether and a modified γ-cyclodextrin", Journal of Chromatography, A (2003), 1000(1-2), 311-324. Publisher: Elsevier Science zitiert Trennmethoden durch Gaschromatographie von Perfluorodiethern z.B. Sevofluran mittels modifiertem γ-Cyclodextrin.

Aufgabe der vorliegenden Erfindung ist es, niedermolekulare halogenierte Ether, welche als Narkosemittel eingesetzt werden, gegenüber Flüchtigkeit zu stabilisieren.

Diese Aufgabe wird gelöst durch einen Komplex bestehend aus einem Cyclodextrin oder Cyclodextrinderivat und einem niedermolekularen halogenierten Ether im molaren Verhältnis 1:1 oder 2:1 oder 3:1.

Bei den niedermolekularen halogenierten Ethern handelt es sich vorzugsweise um chlorierte und/oder fluorierte Ether mit vier oder weniger C-Atomen, besonders bevorzugt um Sevofluran, Isofluran oder Desfluran.

Bei dem Cyclodextrin handelt es sich bevorzugt um ein alpha-, beta- oder gamma-Cyclodextrin. Derartige Cyclodextrine sind beispielsweise unter der Bezeichnung CAVAMAX^{®} W6 Pharma, CAVAMAX^{®} W7 Pharma oder CAVAMAX^{®} W8 Pharma käuflich erhältlich.

Bei dem Cyclodextrinderivat handelt es sich vorzugsweise um ein methyl-, sufobutylether- und hydroxypropyl Derivat eines alpha-, beta- oder gamma- Cyclodextrins.

Derartige Derivate sind beispielsweise unter der Bezeichnung CAVASOL^{®} W7 HP Pharma, CAVASOL^{®} W7 M Pharma, CAVASOL^{®} W8 HP Pharma, CAVITRON^{®} W7 HP5, CAVITRON^{®} W7 HP7 bei der Wacker Chemie AG oder unter der Bezeichnung Captisol von Cydex, bzw KLEPTOSE^{®} KLEPTOSE^{®} 10, KLEPTOSE^{®} 200F, KLEPTOSE^{®} PC, KLEPTOSE^{®} DC von Roquette käuflich erhältlich.

Der erfindungsgemäße Komplex eignet sich als Schlafmittel, denn er ermöglicht eine kontrollierte bedarfsgerechte Freisetzung des Narkosemittels.

Die Erfindung ermöglicht somit erstmals die Verwendung eines komplexierten niedermolekularen halogenierten Ethers, vorzugsweise eines komplexierten halogenierten Ethers mit vier oder weniger C-Atomen, besonders bevorzugt die Verwendung von komplexiertem Sevofluran, Isofluran oder Desfluran als Schlafmittel.

Aufgrund des literaturbekannten Einsatzes von unkomplexierten Cyclodextrinen bzw. deren Derivaten bei Trennmethoden durch Gaschromatographie von Perfluorodiethern oder als Lösungsverstärker bzw. von Komplexen von Cyclodextrinpolymeren war es keineswegs ersichtlich, dass mit alpha-, beta- sowie gamma-Cyclodextrinen sowie deren methyl-, sufobutyl- und hydroxypropyl-Derivaten die Stabilität der vergleichsweise sehr leichtflüchtigen Narkosemittel gegen Flüchtigkeit durch Komplexbildung zu verbessern ist bzw. eine komplett neuartige Darreichungsform zu generieren ist. Bei den literaturbekannten Trennmethoden ist eine hohe Komplexbildung von Nachteil, da der zu trennende Stoff mit dem Cyclodextrin keinen festen Komplex bilden soll. Vielmehr wird der Fachmann von dem Ansatz der vorliegenden Erfindung weggeführt, für die niedermolekularen Flurane im besonderen Sevofluran über die Bildung eines 1:1 bzw. 2:1 bzw. 3:1-Cyclodextrin-Komplexes (Cyclodextrin zu halogeniertem Ether) von alpha-, beta- sowie gamma-Cyclodextrinen sowie deren methyl-, sulfobutylether- und hydroxypropyl-Derivaten die erhoffte Stabilitätserhöhung zu erreichen, da aufgrund der Anwendung als Trennmethode keine definierte Komplexbildung von hoher Stabilität suggeriert wird. In keinem Dokument wird die Stabilisierung eines Narkosemittels gegen Flüchtigkeit, bzw. eine feste als Tablette oder wässrige, z.B. gel-artige Zubereitung (Einschlafmittel) mittels eines Komplexes aus alpha-, beta- oder gamma-Cyclodextrin sowie deren methyl-, sulfobutylether- und hydroxypropyl-Derivaten und Sevofluran, Isofluran oder Desfluran im Verhältnis 1:1 bzw. 2:1 bzw. 3:1 beschrieben.

Überraschend wurde gefunden, dass alpha-, beta- sowie gamma-Cyclodextrine sowie deren methyl-, sulfobutylether- und hydroxypropyl-Derivate mit Sevofluran im Molverhältnis 1:1, 2:1 und 3:1 Komplexe bilden. Der 1:1-Komplex mit methyl- und hydroxypropyl beta-Cyclodextrin sowie der 2:1 Komplex mit gamma-Cyclodextrin zeigt eine gegenüber einer entsprechenden physikalische Mischung der entsprechenden Cyclodextrine mit Sevofluran deutlich erhöhte Stabilität. Im Verhältnis 1:1 bzw. 2:1 oder 3:1 bleibt der Gehalt an Sevofluran in den erfindungsgemäßen 1:1 bzw. 2:1 und 3:1-Komplexen von alpha-, betasowie gamma-Cyclodextrinen sowie deren methyl-, sulfobutylether- und hydroxypropyl-Derivaten und Sevofluran nach Lagerung bei Raumtemperatur (RT), worunter in vorliegender Anmeldung 23°C zu verstehen sind, konstant. Der Wirkstoff ist damit beispielsweise in wässrigen sowie festen Zubereitungen pharmazeutischer Produkte im gewünschten Maß verfügbar.

Die Erfindung betrifft ferner ein Verfahren, welches es ermöglicht, Sevofluran-Komplexe mit alpha-, beta- und gamma-Cyclodextrinen sowie deren methyl-, sulfobutylether- und hydroxypropyl-Derivaten herzustellen.

Dieses Verfahren ist dadurch gekennzeichnet, dass eine wässrige Zubereitung eines alpha-, beta- oder gamma-Cyclodextrins oder eines Methyl-, Sulfobutylether- oder Hydroxypropyl-Derivats dieser Cyclodextrine in einer Konzentration zwischen 2 - 80 Gewichts-% Cyclodextrin mit einem halogenierten Ether in einem Gewichtsverhältnis von Zubereitung zu halogenierten Ether zwischen 4:1 und 35:1 gerührt oder geknetet wird, wobei zu Beginn die wässrige Zubereitung des Cyclodextrins bzw. des Cyclodextrinderivats vorgelegt wird und anschließend der halogenierte Ether zugegeben wird.

Nur so kann eine quantitative Komplexierung erzielt werden.

Als vorteilhaft hat sich die Herstellung der Komplexe aus einer konzentrierten, wässrigen Cyclodextrin-Zubereitung einer Konzentration zwischen 4-70 Gewichts-% erwiesen.

Die Reaktionstemperatur liegt üblicherweise bei 0°C - 80°C. Bevorzugt wird bei 20 - 60 °C, besonders bevorzugt bei 25 - 50 °C gearbeitet. Die Reaktionsdauer hängt von der Temperatur ab und liegt zwischen 10 Minuten und 3 Tagen. Bevorzugt ist eine Reaktionszeit von 1 bis 24 Stunden. Die Komplexierung erfolgt in der Regel unter Normaldruck.

Durch die Verfahrensführung bei der Herstellung werden für die Komplexbildung vorteilhafte Konzentrationen und Konzentrationsverhältnisse erzielt.

Die erfindungsgemäßen Komplexe können durch Filtration, Zentrifugation oder Trocknung aus dem Reaktionsansatz isoliert werden. Sie können durch Mahlen, Sieben, Sichten, Granulieren oder Tablettieren aufbereitet werden. Derartige Verfahren sind für Cyclodextrinkomplexe im Stand der Technik bekannt. Sie werden vorzugsweise in der jeweils bekannten Art und Weise durchgeführt, da es sich bei dem Komplex um ein stabiles Pulver handelt.

Weitere Herstellungsmöglichkeiten umfassen die Komplexierung mit Hilfe von superkritischem CO₂ als Lösungsmittel. Hier wird der halogenierte Ether in superkritischem CO₂ gelöst und über unkomplexiertes CD geleitet, vorzugsweise in den molaren Verhältnissen 1:1, 2:1 oder 3:1 (CD zu halogeniertem Ether). Nach einer ausreichenden Kontaktzeit von 5 min bis 72 h bilden sich die vorher beschriebenen Komplexe. Durch das superkritische Lösungsmittel ist keine weitere Trocknung notwendig.

Die wasserlöslichen Komplexe können nach oben beschriebener Isolierung aber auch direkt, d.h. in Form der erfindungsgemäß erhaltenen Reaktionsmischung, als Schlafmittel verwendet werden.

Stabilitätstests zeigen, dass die Stabilisierung nur bei einer Komplexbildung auftritt. Physikalische Mischungen zeigen dieses Verhalten nicht (Beispiel 6). Auch eine physikalische Mischung von Cyclodextrin und einem Fluran in Wasser führt zu keiner Stabilisierung, der Flurangehalt ist in kürzester Zeit auf 0% (siehe Beispiel 8).
Eine stabile neuartige Darreichungsform von niedermolekularen halogenierten Ethern wird somit nur durch einen erfindungsgemäßen Komplex ermöglicht.

Trotz der hohen Stabilität des trockenen Komplexes kann durch Zugabe von Wasser, wässrigen Lösungen sowie diverser Körperflüssigkeiten eine Freisetzung des Aktivstoffes (z.B. Sevofluran, Isofluran oder Desfluran) erreicht werden (Beispiel 7). Durch den Überschuss an Wasser kann über einen controlledrelease-Effekt der halogenierte Ether abgegeben werden. Trotz der hohen Stabilität der erfindungsgemäßen Komplexe ist ein Wiederfreisetzen des verkapselten Narkosegases in wässriger Lösung problemlos möglich.

Durch den Wasserüberschuss beim Auflösen kann sich der Komplex wieder trennen und das Narkosegas freigesetzt werden. Somit lässt sich eine Nutzung als Schlaftablette oder lokales Anaesthetikum (Anästhtika zur lokalen Betäubung) erst durch einen erfindungsgemäßen Komplex/eine erfindungsgemäße Zusammensetzung realisieren. Bisher wurde der niedermolekulare halogenierte Ether als Narkosemittel nur gasförmig eingesetzt.

Eine weitere Aufgabe der Erfindung war die Bereitstellung einer unter Normbedingungen (273,15 K, 1 bar) flüssigen oder festen pharmazeutischen Formulierung enthaltend niedermolekularen, halogenierten Ether, der über einen Zeitraum von mindestens 90 Tagen stabil in der pharmazeutischen Formulierung vorliegt.

Diese Aufgabe wird gelöst durch eine unter Normbedingungen (273,15 K, 1 bar) flüssige oder feste pharmazeutische Formulierung enthaltend einen erfindungsgemäßen Komplex.

Bei der pharmazeutischen Formulierung handelt es sich vorzugsweise um eine Tablette, ein Dragee oder eine wässrigen Zubereitung (z.B. Auflösen des Komplexes oder einer den Komplex enthaltenden Tablette in Wasser, Getränken oder anderen Flüssigkeiten) enthaltend einen erfindungsgemäßen Komplex.

Die Erfindung betrifft somit ebenfalls ein Spray, ein Aerosol, eine wässrigen Zubereitung wie z.B. eine Lösung, ein Gel, eine Feststoffzubereitung wie eine Tablette, ein Dragee, oder ein Puder, dadurch gekennzeichnet, dass diese Formulierungen einen erfindungsgemäßen Komplex enthalten.

Die pharmazeutische Formulierung enthält neben einem erfindungsgemäßen Komplex vorzugsweise ein galenisches Hilfsmittel, wie sie bei Sprays, Aerosolen, wässrigen pharmazeutischen Zubereitung wie z.B. einer Lösung, einem Gel, einer Feststoffzubereitung wie einer Tablette, einem Dragee, oder einem Puder verwendet werden.

Derartige galenische Hilfsmittel sind für Tabletten oder Dragees beispielsweise
als Fließmittel: hochdisperses Siliciumdioxid oder Talkum; als Binde- und Sprengmittel: Cellulose, besonders mikrokristalline Cellulose oder mikrofeine Cellulose, Cellulosederivate wie z.B. HPMC und HPC, sowie die Polyvinylpyrrolidon (PVP-Kollidone); als Schmiermittel: Magnesiumstearat.

Durch Zusatz derartiger Hilfsmittel werden im Pulvergemisch die zur Tablettierung notwendigen Eigenschaften verbessert und die Eigenschaften der fertigen Tablette modifiziert. Durch Zusatz von Fließmittel werden die Fließeigenschaften des Haufwerkes verbessert. Durch Binde- und Sprengmittel wird das Verpressen zu haltbaren Tabletten sowie der spätere Zerfall im Magen-Darm-Trakt verbessert.

Als galenische Hilfsmittel für pharmazeutische Formulierungen wie Gels können z.B. Propylenglycol, Polyethylenglycol, Hydroxyethylcellulose,Carboxymethylcellulose, Fettalkohole, Natriumchlorid und Wasser verwendet werden.

Vorteile der erfindungsgemäßen pharmazeutischen Formulierungen sind die genaue Dosierbarkeit und die einfache Möglichkeit der Einnahme. Mit der erfindungsgemäßen pharmazeutischen Formulierung wird erstmal ein Einschlafmittel ohne "Hang-over-Effekt", welches z.B. bei Langstreckenflügen erwünscht ist, zur Verfügung gestellt.

Die erfindungsgemäße Formulierung zeichnet sich zudem durch eine hohe Stabilität des Wirkstoffes in der Arzneiform, eine lange Haltbarkeit des Arzneimittels, eine gute Verpackungs-und Transportmöglichkeit sowie eine billige und massenhafte Produktionsmöglichkeit auf geeigneten Maschinen aus. Die Direkttablettierung - Verpressen von Pulvern mit oder ohne Zusatz von Hilfsstoffen - von flüchtigen flüssigen Narkosemitteln, besonders bevorzugt Sevofluran, wird erstmals durch Einsatz der erfindungsgemäßen Komplexe möglich.

Eine weitere Anwendung der erfindungsgemäßen Komplexe / Formulierungen sind Lokalanästhetika, welche topikal, transdermal, mucosal, buccal, rektal, vaginal, subkutan und intrathecal und epidural appliziert werden können. Der Einsatzzweck kann sowohl chronische als auch akute Schmerzen umfassen. Der Vorteil der erfindungsgemäßen Komplexe ist deren schnelle analgetische Wirkung sowie die einfache Einarbeitung in die entsprechenden Darreichungsformen (wie u.a. Pflaster, Patches, Zäpfchen, Cremes, Gele u.v.m.). Durch die Stabilisierung der flüchtigen Verbindungen wird eine Applizierbarkeit in diesen Formen erst möglich.

Eine weitere Anwendung stellt die Anästhesie von Tieren dar. Durch die leichte Flüchtigkeit sind Sevofluran, Isofluran und Desfluran nur schwer bei Tieren zu applizieren. Durch die feste Form können diese Anästhetika leicht verabreicht werden, entweder direkt oder im Futter.
Fig. 1 zeigt die Lagerstabilität von Sevofluran als 1:1 und 2:1 gamma-Cyclodextrin-Komplex und 1:1 und 2:1 Methyl-beta-Cyclodextrinkomplex bzw. eine Mischung dieser Komplexe bei Raumtemperatur.
Fig. 2 zeigt zeigt die Lagerstabilität von Sevofluran als 1:1 und 2:1 gamma-Cyclodextrin-Komplex und 1:1 und 2:1 Methyl-beta-Cyclodextrinkomplex bzw. eine Mischung dieser Komplexe bei 40°C.
Fig. 3 zeigt das Releaseverhalten eines W7M-Cyclodextrin-Sevofluran-Komplexes in einer 30%-igen wässrigen Lösung bei offener Lagerung (Freisetzung von Sevofluran aus dem Komplex) bei 23°C und 60 °C.

Die folgenden Beispiele dienen der weiteren Erläuterung der Erfindung:
Die verwendeten Einsatzstoffe waren:
   Sevofluran der Firma Baxter (100%, Molekulargewicht 200,1 g/mol)
   Isofluran der Firma Baxter (100%, Molekulargewicht 184,5 g/mol)
   Desfluran der Firma Baxter (100%, Molekulargewicht 168 g/mol) Methyl-beta-Cyclodextrin, durchschnittliches Molekulargewicht 1310 g/mol
   alpha-Cyclodextrin, Molekulargewicht 975g/mol
   beta-Cyclodextrin, Molekulargewicht 1135g/mol
   gamma-Cyclodextrin, Molekulargewicht 1297g/mol Hydroxypropyl-beta-Cyclodextrin, Molekulargewicht 1400 g/mol Hydroxypropyl-gamma-Cyclodextrin, Molekulargewicht 1574 g/mol Sulfobutylether-Beta-Cyclodextrin, Molekulargewicht 2163 g/mol Die % Angabe in den Beispielen sind Gew.%

### Beispiel 1: Komplexierung von Sevofluran mit Cyclodextrinen und Cyclodextrinderivaten

### 1:1- Alpha-Cyclodextrin-Komplex

48 mmol bzw. 46,8g alpha-Cyclodextrin (Wassergehalt 2%) wurden mit 30g H₂O unter Rühren vermischt und auf 50°C erwärmt. Während des Erwärmens auf 50°C wurden 48 mmol bzw. 9,7 g Sevofluran zugegeben. Der Ansatz wurde 2h bei 50°C gehalten, gerührt, und im Anschluss unter Vakuum bei 35°C getrocknet.
Ausbeute: 51g bzw. 90%, Feuchte: 5%, Gehalt an Sevofluran über Kernspinresonanz-Spektroskopie (NMR) bestimmt: 2%

### 2:1- Alpha-Cyclodextrin Komplex

48 mmol bzw. 46,8g methyl-beta-Cyclodextrin (Wassergehalt 2%) wurden mit 30g H₂O unter Rühren vermischt und auf 50°C erwärmt. Während des Erwärmens auf 50°C wurden 4 mmol bzw. 4,8 g Sevofluran zugegeben. Der Ansatz wurde 2h bei 50°C gehalten, gerührt und im Anschluss unter Vakuum bei 35°C getrocknet. Ausbeute: 46g bzw. 89%, Feuchte: 6%, Gehalt an Sevofluran über NMR bestimmt: 2%

### 1:1- Beta-Cyclodextrin-Komplex

48 mmol bzw. 54,5g Beta-Cyclodextrin (Wassergehalt 2%) wurden mit 30g H₂O unter Rühren vermischt und auf 50°C erwärmt. Während des Erwärmens auf 50°C wurden 48 mmol bzw. 9,7 g Sevofluran zugegeben. Der Ansatz wurde 2h bei 50°C gehalten, gerührt und im Anschluss unter Vakuum bei 35°C getrocknet.
Ausbeute: 58g bzw. 90%, Feuchte: 6%, Gehalt an Sevofluran über NMR bestimmt: 5%

### 2:1- Beta-Cyclodextrin-Komplex

48 mmol bzw. 54,5g Beta-Cyclodextrin (Wassergehalt 2%) wurden mit 30g H₂O unter Rühren vermischt und auf 50°C erwärmt. Während des Erwärmens auf 50°C wurden 24 mmol bzw. 4,8 g Sevofluran zugegeben. Der Ansatz wurde 2h bei 50°C gehalten, gerührt und im Anschluss unter Vakuum bei 35°C getrocknet.
Ausbeute: 68g bzw. 92%, Feuchte: 6%, Gehalt an Sevofluran über NMR bestimmt: 2%

### 3:1-Gamma-Cyclodextrin-Komplex

43 mmol bzw. 60g gamma-Cyclodextrin (Wassergehalt 10%) wurden mit 33g H₂O unter Rühren zu einem Cyclodextrinbrei vermischt.

Anschließend wurden 14 mmol bzw. 2,8 g Sevofluran zugegeben. Der Ansatz wurde 1h bei 40°C gehalten, gerührt und im Anschluss bei 23°C getrocknet.
Ausbeute nach 50h lufttrocknen: 61g bzw. 98%, Gehalt an Sevofluran über NMR bestimmt: 4%

### 1:1- Beta-hydroxypropyl-Cyclodextrin-Komplex

48 mmol bzw. 67,2g Beta-hydroxypropyl-Cyclodextrin (Wassergehalt 2%) wurden mit 30g H₂O unter Rühren vermischt und auf 50°C erwärmt. Während des Erwärmens auf 50°C wurden 48 mmol bzw. 9,7 g Sevofluran zugegeben. Der Ansatz wurde 2h bei 50°C gehalten, gerührt und im Anschluss unter Vakuum bei 35°C getrocknet.
Ausbeute: 72g bzw. 94%, Feuchte: 5%, Gehalt an Sevofluran über NMR bestimmt: 12%

### 2:1- Beta-hydroxypropyl-Cyclodextrin-Komplex

48 mmol bzw. 67,2g Beta-hydroxypropyl-Cyclodextrin (Wassergehalt 2%) wurden mit 30g H₂O unter Rühren vermischt und auf 50°C erwärmt. Während des Erwärmens auf 50°C wurden 24 mmol bzw. 4,8 g Sevofluran zugegeben. Der Ansatz wurde 2h bei 50°C gehalten, gerührt und im Anschluss unter Vakuum bei 35°C getrocknet.
Ausbeute: 68g bzw. 94%, Feuchte: 7%, Gehalt an Sevofluran über NMR bestimmt: 7%

### 1:1- Gamma-Hydroxypropyl-Cyclodextrin Komplex

48 mmol bzw. 75,6g Gamma-Hydroxypropyl-Cyclodextrin (Wassergehalt 2%) wurden mit 30g H₂O unter Rühren vermischt und auf 50°C erwärmt. Während des Erwärmens auf 50°C wurden 48 mmol bzw. 9,7 g Sevofluran zugegeben. Der Ansatz wurde 2h bei 50°C gehalten, gerührt und im Anschluss unter Vakuum bei 35°C getrocknet.
Ausbeute: 81g bzw. 95%, Feuchte: 4%, Gehalt an Sevofluran über NMR bestimmt: 11%

### 2:1- Gamma-Hydroxypropyl-Cyclodextrin-Komplex

48 mmol bzw. 75,6g Gamma-Hydroxypropyl-Cyclodextrin (Wassergehalt 2%) wurden mit 30g H₂O unter Rühren vermischt und auf 50°C erwärmt. Während des Erwärmens auf 50°C wurden 24 mmol bzw. 4,8g Sevofluran zugegeben. Der Ansatz wurde 2h bei 50°C gehalten, gerührt und im Anschluss unter Vakuum bei 35°C getrocknet.
Ausbeute: 77g bzw. 96%, Feuchte: 6%, Gehalt an Sevofluran über NMR bestimmt: 6%

### 1:1- Sulfobutylether-beta-Cyclodextrin-Komplex

48 mmol bzw. 103,8g Sulfobutylether-beta-Cyclodextrin wurden mit 30g H₂O unter Rühren vermischt und auf 50°C erwärmt. Während des Erwärmens auf 50°C wurden 48 mmol bzw. 9,7 g Sevofluran zugegeben. Der Ansatz wurde 2h bei 50°C gehalten, gerührt und im Anschluss unter Vakuum bei 35°C getrocknet.
Ausbeute: 109g bzw. 96%, Feuchte: 4%, Gehalt an Sevofluran über NMR bestimmt: 8%

### 2:1- Sulfobutylether-beta-Cyclodextrin-Komplex

48 mmol bzw. 103,8g Sulfobutylether-beta-Cyclodextrin wurden mit 30g H₂O unter Rühren vermischt und auf 50°C erwärmt. Während des Erwärmens auf 50°C wurden 24 mmol bzw. 4,8 g Sevofluran zugegeben. Der Ansatz wurde 2h bei 50°C gehalten, gerührt und im Anschluss unter Vakuum bei 35°C getrocknet.
Ausbeute: 103g bzw. 95%, Feuchte: 4%, Gehalt an Sevofluran über NMR bestimmt: 4%

### Beispiel 2: Komplexierung von Isofluran mit Cyclodextrinen und Cyclodextrinderivaten

### 1:1-gamma-Cyclodextrin-Komplex

48 mmol bzw. 62g gamma-Cyclodextrin (Wassergehalt 10%) wurden mit 33g H₂O unter Rühren zu einem Cyclodextrinbrei vermischt. Anschließend wurden 48 mmol bzw. 8,8 g Isofluran zugegeben. Der Ansatz wurde 1h bei 40°C gehalten, gerührt und im Anschluss bei RT getrocknet.
Ausbeute nach 50h lufttrocknen: 69g bzw. 97%, Gehalt an Isofluran über NMR bestimmt: 11%

### 2:1-gamma-Cyclodextrin-Komplex

48 mmol bzw. 62g gamma-Cyclodextrin (Wassergehalt 10%) wurden mit 33g H₂O unter Rühren zu einem Cyclodextrinbrei vermischt. Anschließend wurden 24 mmol bzw. 4,4 g Isofluran zugegeben. Der Ansatz wurde 1h bei 40°C gehalten, gerührt und im Anschluss bei RT getrocknet.

Ausbeute nach 50h lufttrocknen: 64g bzw. 96%, Gehalt an Isofluran über NMR bestimmt: 6%

### 3:1-Gamma-Cyclodextrin-Komplex

48 mmol bzw. 62g gamma-Cyclodextrin (Wassergehalt 10%) wurden mit 33g H2O unter Rühren zu einem Cyclodextrinbrei vermischt. Anschließend wurden 16 mmol bzw. 2,9 g Isofluran zugegeben. Der Ansatz wurde 1h bei 40°C gehalten, gerührt und im Anschluss bei RT getrocknet.
Ausbeute nach 50h lufttrocknen: 63g bzw. 99%, Gehalt an Isofluran über NMR bestimmt: 4%

### 1:1- Methyl-beta-Cyclodextrin-Komplex

48 mmol bzw. 62,9g methyl-beta-Cyclodextrin (Wassergehalt 2%) wurden mit 30g H₂O unter Rühren vermischt und auf 50°C erwärmt. Während des Erwärmens auf 50°C wurden 48 mmol bzw. 8,8 g Isofluran zugegeben. Der Ansatz wurde 2h bei 50°C gehalten, gerührt und im Anschluss unter Vakuum bei 35°C getrocknet. Ausbeute: 65g bzw. 91%, Feuchte: 5%, Gehalt an Isofluran über NMR bestimmt: 12%

### 2:1-Methyl-beta-Cyclodextrin-Komplex

48 mmol bzw. 62,9g Methyl-beta-Cyclodextrin (Wassergehalt 2%) wurden mit 30g H₂O unter Rühren vermischt und auf 50°C erwärmt. Während des Erwärmens auf 50°C wurden 24 mmol bzw. 4,4 g Isofluran zugegeben. Der Ansatz wurde 2h bei 50°C gehalten, gerührt und im Anschluss unter Vakuum bei 35°C getrocknet.
Ausbeute: 61g bzw. 91% Feuchte: 5%, Gehalt an Isofluran über NMR bestimmt: 6 %

### 1:1- Beta-hydroxypropyl-Cyclodextrin-Komplex

48 mmol bzw. 67,2g Beta-hydroxypropyl-Cyclodextrin (Wassergehalt 2%) wurden mit 30g H₂O unter Rühren vermischt und auf 50°C erwärmt. Während des Erwärmens auf 50°C wurden 48 mmol bzw. 8,8 g Isofluran zugegeben. Der Ansatz wurde 2h bei 50°C gehalten, gerührt und im Anschluss unter Vakuum bei 35°C getrocknet.
Ausbeute: 73g bzw. 96%, Feuchte: 4%, Gehalt an Isofluran über NMR bestimmt: 11%

### 2:1- Beta-hydroxypropyl-Cyclodextrin-Komplex

48 mmol bzw. 67,2g Beta-hydroxypropyl-Cyclodextrin (Wassergehalt 2%) wurden mit 30g H₂O unter Rühren vermischt und auf 50°C erwärmt. Während des Erwärmens auf 50°C wurden 24 mmol bzw. 4,4 g Isofluran zugegeben. Der Ansatz wurde 2h bei 50°C gehalten, gerührt und im Anschluss unter Vakuum bei 35°C getrocknet.
Ausbeute: 67g bzw. 93%, Feuchte: 6%, Gehalt an Isofluran über NMR bestimmt: 5%

### 1:1- Gamma-Hydroxypropyl-Cyclodextrin-Komplex

48 mmol bzw. 75,6g Gamma-Hydroxypropyl-Cyclodextrin (Wassergehalt 2%) wurden mit 30g H₂O unter Rühren vermischt und auf 50°C erwärmt. Während des Erwärmens auf 50°C wurden 48 mmol bzw. 8,8 g Isofluran zugegeben. Der Ansatz wurde 2h bei 50°C gehalten, gerührt und im Anschluss unter Vakuum bei 35°C getrocknet.
Ausbeute: 78g bzw. 93%, Feuchte: 5%, Gehalt an Isofluran über NMR bestimmt: 10%

### 2:1- Gamma-Hydroxypropyl-Cyclodextrin-Komplex

48 mmol bzw. 75,6g Gamma-Hydroxypropyl-Cyclodextrin (Wassergehalt 2%) wurden mit 30g H₂O unter Rühren vermischt und auf 50°C erwärmt. Während des Erwärmens auf 50°C wurden 24 mmol bzw. 4,4 g Isofluran zugegeben. Der Ansatz wurde 2h bei 50°C gehalten, gerührt und im Anschluss unter Vakuum bei 35°C getrocknet.
Ausbeute: 76g bzw. 95%, Feuchte: 5%, Gehalt an Isofluran über NMR bestimmt: 5%

### 1:1- Sulfobutylether-beta-Cyclodextrin-Komplex

48 mmol bzw. 103,8g Sulfobutylether-beta-Cyclodextrin wurden mit 30g H₂O unter Rühren vermischt und auf 50°C erwärmt. Während des Erwärmens auf 50°C wurden 48 mmol bzw. 8,8 g Isofluran zugegeben. Der Ansatz wurde 2h bei 50°C gehalten, gerührt und im Anschluss unter Vakuum bei 35°C getrocknet.
Ausbeute: 109g bzw. 97%, Feuchte: 3%, Gehalt an Isofluran über NMR bestimmt: 7%

### 2:1- Sulfobutylether-beta-Cyclodextrin-Komplex

48 mmol bzw. 103,8g Sulfobutylether-beta-Cyclodextrin wurden mit 30g H₂O unter Rühren vermischt und auf 50°C erwärmt. Während des Erwärmens auf 50°C wurden 24 mmol bzw. 4,4 g Isofluran zugegeben. Der Ansatz wurde 2h bei 50°C gehalten, gerührt und im Anschluss unter Vakuum bei 35°C getrocknet.
Ausbeute: 104g bzw. 96%, Feuchte: 3%, Gehalt an Isofluran über NMR bestimmt: 4%

### Beispiel 3: Komplexierung von Desfluran mit Cyclodextrinen und Cyclodextrinderivaten

### 1:1-gamma-Cyclodextrin-Komplex

48 mmol bzw. 62g gamma-Cyclodextrin (Wassergehalt 10%) wurden mit 33g H₂O unter rühren zu einem Cyclodextrinbrei vermischt. Anschließend wurden 48 mmol bzw. 8,1 g Desfluran zugegeben. Der Ansatz wurde 1h bei 40°C gehalten, gerührt und im Anschluss bei RT getrocknet.
Ausbeute nach 50h lufttrocknen: 66g bzw. 98%, Feuchte nach 1 h Trocknen: 36% H₂O, Gehalt an Desfluran: 11%

### 2:1-gamma-Cyclodextrin Komplex

48 mmol bzw. 62g gamma-Cyclodextrin (Wassergehalt 10%) wurden mit 33g H₂O unter Rühren zu einem Cyclodextrinbrei vermischt. Anschließend wurden 24 mmol bzw. 4,0 g Desfluran zugegeben. Der Ansatz wurde 1h bei 40°C gehalten, gerührt und im Anschluss bei RT getrocknet.

Ausbeute nach 50h lufttrocknen: 62g bzw. 97%, Gehalt an Desfluran : 6%

### 3:1-Gamma-Cyclodextrin-Komplex

48 mmol bzw. 62g gamma-Cyclodextrin (Wassergehalt 10%) wurden mit 33g H₂O unter Rühren zu einem Cyclodextrinbrei vermischt. Anschließend wurden 16 mmol bzw. 2,7 g Desfluran zugegeben. Der Ansatz wurde 1h bei 40°C gehalten, gerührt und im Anschluss bei RT getrocknet.
Ausbeute nach 50h lufttrocknen: 61g bzw. 98%, Gehalt an Desfluran: 4%

### 1:1- Methyl-beta-Cyclodextrin-Komplex

48 mmol bzw. 64,7g methyl-beta-Cyclodextrin (Wassergehalt 2%) wurden mit 30g H₂O unter Rühren vermischt und auf 50°C erwärmt. Während des Erwärmens auf 50°C 48 mmol bzw. 8,1 g Desfluran zugegeben. Der Ansatz wurde 2h bei 50°C gehalten, gerührt und im Anschluss unter Vakuum bei 35°C getrocknet. Ausbeute: 68g bzw. 94%, Feuchte: 6%, Gehalt an Desfluran über NMR bestimmt: 11%

### 2:1-Methyl-beta-Cyclodextrin-Komplex

48 mmol bzw. 64,7g Methyl-beta-Cyclodextrin (Wassergehalt 2%) wurden mit 30g H₂O unter Rühren vermischt und auf 50°C erwärmt. Während des Erwärmens auf 50°C wurden 24 mmol bzw. 4,0 g Desfluran zugegeben. Der Ansatz wurde 2h bei 50°C gehalten, gerührt und im Anschluss unter Vakuum bei 35°C getrocknet.
Ausbeute: 63g bzw. 92% Feuchte: 6%
Gehalt an Desfluran über NMR bestimmt: 5 %

### 1:1- Beta-hydroxypropyl-Cyclodextrin-Komplex

48 mmol bzw. 67,2g Beta-hydroxypropyl-Cyclodextrin (Wassergehalt 2%) wurden mit 30g H₂O unter Rühren vermischt und auf 50°C erwärmt. Während des Erwärmens auf 50°C wurden 48 mmol bzw. 8,1 g Desfluran zugegeben. Der Ansatz wurde 2h bei 50°C gehalten, gerührt und im Anschluss unter Vakuum bei 35°C getrocknet.
Ausbeute: 70g bzw. 93%, Feuchte: 4%, Gehalt an Desfluran über NMR bestimmt: 10%

### 2:1- Beta-hydroxypropyl-Cyclodextrin-Komplex

48 mmol bzw. 67,2g Beta-hydroxypropyl-Cyclodextrin (Wassergehalt 2%) wurden mit 30g H₂O unter Rühren vermischt und auf 50°C erwärmt. Während des Erwärmens auf 50°C wurden 24 mmol bzw. 4,0 g Desfluran zugegeben. Der Ansatz wurde 2h bei 50°C gehalten, gerührt und im Anschluss unter Vakuum bei 35°C getrocknet.
Ausbeute: 68g bzw. 95%, Feuchte: 5%, Gehalt an Desfluran über NMR bestimmt: 5%

### 1:1- Gamma-Hydroxypropyl-Cyclodextrin-Komplex

48 mmol bzw. 75,6g Gamma-Hydroxypropyl-Cyclodextrin (Wassergehalt 2%) wurden mit 30g H₂O unter Rühren vermischt und auf 50°C erwärmt. Während des Erwärmens auf 50°C wurden 48 mmol bzw. 8,1g Desfluran zugegeben. Der Ansatz wurde 2h bei 50°C gehalten, gerührt und im Anschluss unter Vakuum bei 35°C getrocknet.
Ausbeute: 79g bzw. 94%, Feuchte: 6%, Gehalt an Desfluran über NMR bestimmt: 9%

### 2:1- Gamma-Hydroxypropyl-Cyclodextrin-Komplex

48 mmol bzw. 75,6g Gamma-Hydroxypropyl-Cyclodextrin (Wassergehalt 2%) wurden mit 30g H₂O unter Rühren vermischt und auf 50°C erwärmt. Während des Erwärmens auf 50°C 24 mmol bzw. 4,0g Desfluran zugegeben. Der Ansatz wurde 2h bei 50°C gehalten, gerührt und im Anschluss unter Vakuum bei 35°C getrocknet. Ausbeute: 75g bzw. 95%, Feuchte: 5%, Gehalt an Desfluran über NMR bestimmt: 5%

### 1:1- Sulfobutylether-beta-Cyclodextrin-Komplex

48 mmol bzw. 103,8g Sulfobutylether-beta-Cyclodextrin wurden mit 30g H₂O unter Rühren vermischt und auf 50°C erwärmt. Während des Erwärmens auf 50°C wurden 48 mmol bzw. 8,1 g Desfluran zugegeben. Der Ansatz wurde 2h bei 50°C gehalten, gerührt und im Anschluss unter Vakuum bei 35°C getrocknet.
Ausbeute: 106g bzw. 95%, Feuchte: 4%, Gehalt an Desfluran über NMR bestimmt: 7%

### 2:1- Sulfobutylether-beta-Cyclodextrin-Komplex

48 mmol bzw. 103,8g Sulfobutylether-beta-Cyclödextrin wurden mit 30g H₂O unter Rühren vermischt und auf 50°C erwärmt. Während des Erwärmens auf 50°C wurden 24 mmol bzw. 4,0 g Desfluran zugegeben. Der Ansatz wurde 2h bei 50°C gehalten, gerührt und im Anschluss unter Vakuum bei 35°C getrocknet.
Ausbeute: 103g bzw. 96%, Feuchte: 5%, Gehalt an Desfluran über NMR bestimmt: 4%

### Beispiel 4: Komplexierung von Sevofluran mit methyl-beta-Cyclodextrin für einen Vergleich der Lagerstabilität

### 1:1- Methyl-beta-Cyclodextrin-Komplex

48 mmol bzw. 64,7g methyl-beta-Cyclodextrin (Wassergehalt 2%) wurden mit 30g H₂O unter Rühren vermischt und auf 50°C erwärmt. Während des Erwärmens auf 50°C wurden 48 mmol bzw. 9,7 g Sevofluran zugegeben. Der Ansatz wurde 2h bei 50°C gehalten, gerührt und im Anschluss unter Vakuum bei 35°C getrocknet. Ausbeute: 68g bzw. 92%, Feuchte: 6%, Gehalt an Sevofluran über NMR bestimmt: 12%

### 2:1-Methyl-beta-Cyclodextrin-Komplex

48 mmol bzw. 64,7g Methyl-beta-Cyclodextrin (Wassergehalt 2%) wurden mit 30g H₂O unter Rühren vermischt und auf 50°C erwärmt. Während des Erwärmens auf 50°C wurden 24 mmol bzw. 4,8 g Sevofluran zugegeben. Der Ansatz wurde 2h bei 50°C gehalten, gerührt und im Anschluss unter Vakuum bei 35°C getrocknet. Ausbeute: 62,2g bzw. 90% Feuchte: 7%
Gehalt an Sevofluran über NMR bestimmt: 6,1 %

### Physikalische Mischung 1:1 Methyl-beta-Cyclodextrin /Sevofluran

Für die physikalische Mischung wurden 48 mmol bzw. 64,7g Methyl-beta-Cyclodextrin (Wassergehalt 2,2%) und 48 mmol bzw. 9,7 g Sevofluran mit Reibschale und Pistill homogenisiert und der Gehalt an Sevofluran unmittelbar nach der Herstellung ermittelt.
Gehalt an Sevofluran über NMR bestimmt: 0 %

### Physikalische Mischung 2:1 Methyl-beta-Cyclodextrin /Sevofluran

Für die physikalische Mischung wurden 48 mmol bzw. 64,7g Methyl-β-Cyclodextrin (Wassergehalt 2,2%) und 24 mmol bzw. 4,8g Sevofluran mit Reibschale und Pistill homogenisiert und der Gehalt an Sevofluran unmittelbar nach der Herstellung ermittelt.
Gehalt an Sevofluran über NMR bestimmt: 0%

### Beispiel 5: Komplexierung von Sevofluran mit gamma-Cyclodextrin für einen Vergleich der Lagerstabilität

### 1:1-gamma-Cyclodextrin Komplex

43 mmol bzw. 60g gamma-Cyclodextrin (Wassergehalt 10%) wurden mit 33g H₂O unter Rühren zu einem Cyclodextrinbrei vermischt. Anschließend wurden 43 mmol bzw. 8,5 g Sevofluran zugegeben. Der Ansatz wurde 1h bei 40°C gehalten, gerührt und im Anschluss bei RT getrocknet.

Ausbeute nach 50h Lufttrocknen: 100g bzw. 98%, Feuchte nach 1 h Trocknen: 36% H₂O, Gehalt an Sevofluran: 8%

### 2:1-gamma-Cyclodextrin Komplex

43 mmol bzw. 60g gamma-Cyclodextrin (Wassergehalt 10%) wurden mit 33g H₂O unter Rühren zu einem Cyclodextrinbrei vermischt.

Anschließend wurden 21 mmol bzw. 4,3 g Sevofluran zugegeben. Der Ansatz wurde 1h bei 40°C gehalten, gerührt und im Anschluss bei RT getrocknet.

Ausbeute nach 50h Lufttrocknen: 97g bzw. 99%, Gehalt an Sevofluran: 4%

### aa) Physikalische Mischung 1:1 gamma-Cyclodextrin/Sevofluran

Für die physikalische Mischung wurden 43 mmol bzw. 60g gamma-Cyclodextrin (Wassergehalt 8%) und 43 mmol bzw. 8,5g Sevofluran mit Reibschale und Pistill homogenisiert und der Gehalt an Sevofluran unmittelbar nach der Herstellung der physikalischen Mischung ermittelt. Ausbeute: 60g bzw. 88%
Gehalt Sevofluran über NMR bestimmt: 0%; Theoretischer Sevofluran-Gehalt 12%;

### bb) Physikalische Mischung 2:1 gamma-Cyclodextrin/Sevofluran

Für die physikalische Mischung wurden 43 mmol bzw. 60g gamma-Cyclodextrin (Wassergehalt 8%) und 21,5 mmol bzw. 4,3g Sevofluran mit Reibschale und Pistill homogenisiert u. der Gehalt an Sevofluran unmittelbar nach der Herstellung der physikalischen Mischung ermittelt.
Ausbeute: 60g bzw. 93%
Gehalt Sevofluran über NMR bestimmt: 0%; Theoretischer Sevofluran-Gehalt 6,7%;

### Beispiel 6: Bestimmung der Lagerstabilität von Sevofluran als 1:1 und 2:1 gamma-Cyclodextrin-Komplex und 1:1 und 2:1 Methyl-beta-Cyclodextrinkomplex bzw. eine Mischung dieser Komplexe

### a) bei Raumtemperatur

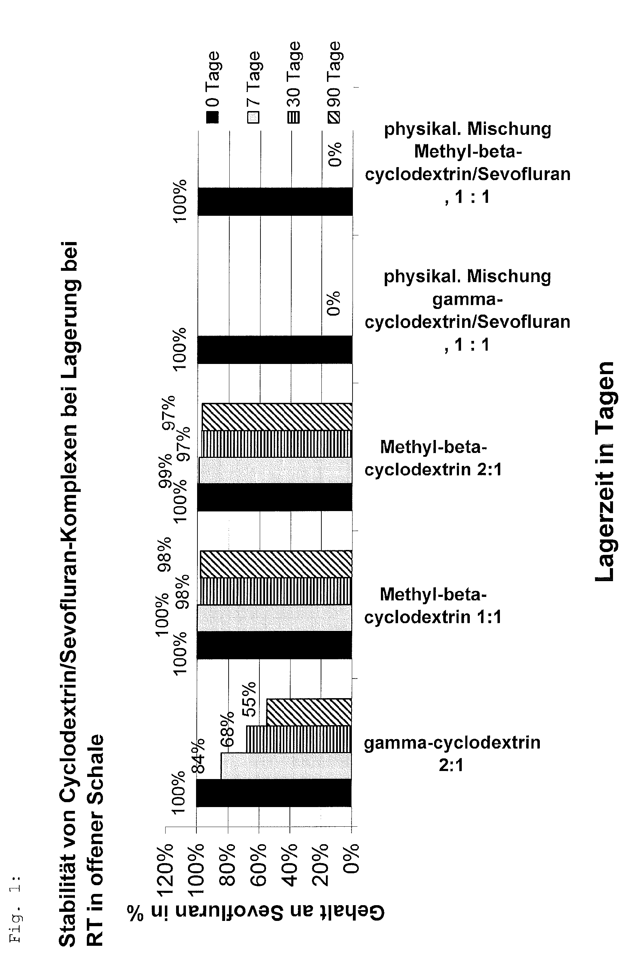

Die in den Beispielen 4 und 5 erhaltenen Produkte wurden bei Raumtemperatur in einem offenen Gefäß (Durchmesser 90mm und eine Schichtdicke von ca. 2 mm) gelagert.
Der Gehalt der Komplexe an Sevofluran wurde über NMR bestimmt. Die Ergebnisse sind in Fig. 1 dargestellt.

### b) bei 40°C gelagert

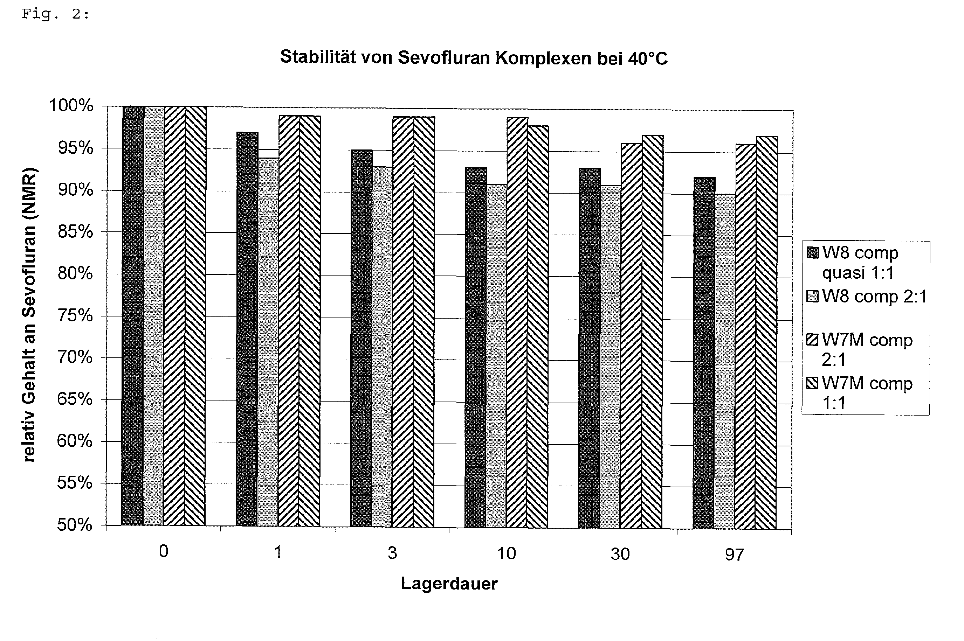

Die in den Beispielen 4 und 5 erhaltenen Produkte wurden bei 40°C im Trockenschrank in einem offenen Gefäß (Durchmesser 90mm und eine Schichtdicke von ca. 2 mm) gelagert.
Der Gehalt der Komplexe an Sevofluran wurde über NMR bestimmt. Die Ergebnisse sind in Fig. 2 dargestellt.

### Beispiel 7: Releaseverhalten des Komplexes in wässrigen Lösungen bei offerner Lagerung am Beispiel eines 30%igen 1:1

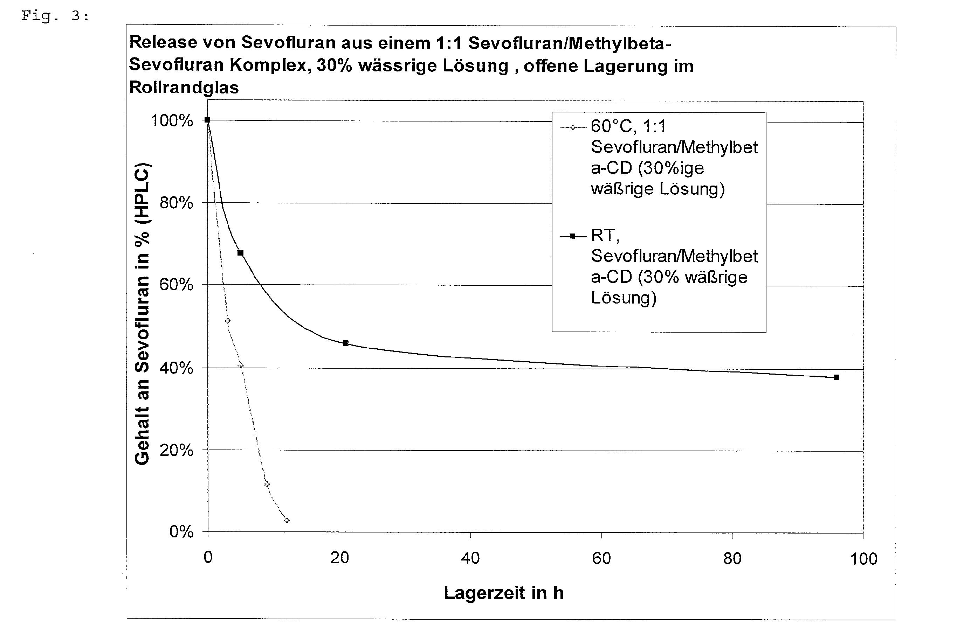

**Methly-beta-(Cavasol W7M)-Cyclodextrin/Sevofluran- Komplexes bei 2 Temperaturen (23°C und 60°C) .**
Der Komplex wurde analog Beispiel 4 hergestellt.
Der Gehalt an Sevofluran in der Lösung wurde mittels HPLC nach jeweils definierten Zeitabständen gemessen.
Die Ergebnisse sind in Fig. 3 dargestellt.
Es findet eine kontrollierte Freisetzung des Komplexes in wässriger Lösung statt.

### Beispiel 8: Physikalische Mischung in Wasser (Vergleichsbeispiel)

### Bestimmung der Lagerstabilität von Sevofluran als 1:1 physi-aklische Mischung mit Methyl-beta-Cyclodextrin.

48 mmol bzw. 62,9g methyl-beta-Cyclodextrin (Wassergehalt 2%) wurden mit 170g H₂O unter Rühren vermischt, danach wurde sofort 48 mmol bzw. 8,8 g Isofluran zugegeben und gerührt. Die resultierende Lösung wurde offen bei RT stehengelassen. Der Gehalt an Sevofluran wurde mittels HPLC nach 24 h gemessen. Es konnte kein Sevoflurangehalt festgestellt werden.

## Patentansprüche

1. Komplex bestehend aus einem Cyclodextrin oder Cyclodextrinderivat und einem niedermolekularen halogeniertem Ether im molaren Verhältnis 1:1 oder 2:1 oder 3:1.

2. Komplex gemäß Anspruch 1, **dadurch gekennzeichnet, dass** das der halogenierte Ether vier oder weniger C-Atome besitzt und es sich dabei bevorzugt um Sevofluran, Isofluran oder Desfluran handelt.

3. Komplex gemäß Anspruch 1 oder 2, **dadurch gekennzeichnet, dass** das Cyclodextrin ein alpha-, beta- oder gamma-Cyclodextrin ist und das Cyclodextrinderivat ein methyl-, sufobutylether- und hydroxypropyl-Derivat eines alpha-, beta- oder gamma-Cyclodextrins ist.

4. Verfahren zur Herstellung eines Komplexes gemäß Anspruch 1, 2 oder 3, **dadurch gekennzeichnet, dass** eine wässrige Zubereitung eines alpha-, beta- oder gamma-Cyclodextrins oder eines Methyl-, Sulfobutylether- oder Hydroxypropyl-Derivats eines alpha-, beta- oder gamma-Cyclodextrins mit einer Konzentration zwischen 2 - 80 Gewichts-% an Cyclodextrin mit einem halogeniertem Ether in einem GewichtsVerhältnis von Cyclodextrin zu halogeniertem Ether zwischen 4:1 und 35:1 gerührt oder geknetet wird, wobei zu Beginn die wässrige Zubereitung des Cyclodextrins bzw. des Cyclodextrinderivats vorgelegt wird und anschließend der halogenierte Ether zugegeben wird.

5. Verfahren gemäß Anspruch 4, **dadurch gekennzeichnet, dass** die Herstellung der Komplexe aus einer konzentrierten, wässrigen Cyclodextrin-Zubereitung einer Konzentration zwischen 4 - 70 Gewichts-% erfolgt.

6. Verfahren gemäß Anspruch 4 oder 5, **dadurch gekennzeichnet, dass** die Reaktionstemperatur bei 0°C - 80°C, bevorzugt bei 20 - 60 °C, besonders bevorzugt bei 25 - 50 °C liegt und die Reaktionsdauer in Abhängigkeit von der Temperatur zwischen 10 Minuten und 3 Tagen, bevorzugt bei 1 bis 24 Stunden liegt und unter einem Druck von einem bar erfolgt.

7. Unter Normbedingungen flüssige oder feste pharmazeutische Formulierung enthaltend einen Komplex gemäß Anspruch 1 bis 3.

8. Pharmazeutische Formulierung gemäß Anspruch 7 in Form einer Tablette, eines Dragees oder einer wässrigen Zubereitung.

9. Pharmazeutische Formulierung gemäß Anspruch 7 oder 8 enthaltend ein galenisches Hilfsmittel.

10. Verwendung eines Komplexes gemäß Anspruch 1 bis 3 oder einer Formulierung gemäß Anspruch 7 bis 9 als Schlafmittel oder Lokalanästhetikum.
